(19) Europäisches Patentamt
European Patent Office
Office européen des brevets

(11) **EP 2 623 156 A2**

(12) **EUROPEAN PATENT APPLICATION**

(43) Date of publication:
**07.08.2013 Bulletin 2013/32**

(51) Int Cl.:
**A61N 7/02** (2006.01)

(21) Application number: **12194787.3**

(22) Date of filing: **29.11.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.01.2012 KR 20120009739**

(71) Applicant: **Samsung Electronics Co., Ltd Gyeonggi-do 443-742 (KR)**

(72) Inventors:
• **Lee, Hyoung-ki**
  **Gyeonggi-do (KR)**
• **Park, Jun-ho**
  **Gyeonggi-do (KR)**
• **Shim, Mun-bo**
  **Gyeonggi-do (KR)**

(74) Representative: **Grootscholten, Johannes A.M.**
**Arnold & Siedsma**
**Sweelinckplein 1**
**2517 GK Den Haag (NL)**

(54) **Method and apparatus for forming multi-focus of ultrasound transducer array**

(57) A method and apparatus for forming a multi-focus of an ultrasound transducer array. Accordingly, by simultaneously forming multiple focal points while minimizing grating lobes in an operation of a High Intensity Focused Ultrasound (HIFU) device, a damage to an area outside a target area, for example damage to organs around a lesion during therapy or other fields of use using the HIFU device may be prevented.

FIG. 1

**Description**

BACKGROUND

1. Field

One or more embodiments of the present disclosure relate to forming an ultrasound focus which may comprise at least one focal point of for instance a High Intensity Focused Ultrasound (HIFU) or other ultrasound device, and more particularly, to an apparatus and method for forming a multi-focus of an ultrasound transducer array.

2. Description of the Related Art

Along with the development of medical science, extended radical surgery, function-preserving surgery, and minimally invasive surgery have been gradually developed for local treatment of tumors. However, recent revolutionary technical advances have further developed noninvasive surgery to introduce a variety of new surgery methods.
An ultrasound wave is mechanical energy having enough directivity to penetrate the human body. The ultrasound wave largely has two effects - a hyperthermal effect and a mechanical effect, through energy accumulation in a tumor that is caused by irradiation of a HIFU wave.
The first effect, that is, the hyperthermal effect, is an effect that causes a temperature to instantaneously increase to over 70°C while a high intensity ultrasound wave focused on a part is partially transduced into thermal energy, resulting in coagulation necrosis in tissue and blood vessels. In tissue subjected to the hyperthermal effect, the temperature is rapidly and instantaneously increased, and therefore thermal diffusion to surrounding tissues does not occur.
The second effect, that is, the mechanical effect due to the ultrasound wave, results in a tissue destruction phenomenon due to cavitation. When a human body is exposed to a high intensity ultrasound wave, liquids in cells change to a gas phase due to a low atmospheric pressure according to a negative pressure of a sound wave, causing generation of microbubbles in the cells. When the microbubbles are large enough to cause a resonance phenomenon, the microbubbles burst suddenly, thereby generating a shock wave having a high pressure that is destructive to tissue.
These two effects occur almost simultaneously, causing changes to the treated tissue that are visible to the naked eye. For example, coagulation necrosis occurs after one or two weeks due to irradiation of an ultrasound wave and thus the treated part is clearly discriminated from its surrounding normal parts and a boundary part between the treated part and the normal parts feels hard.
However, when a tumor is treated, damage to normal tissue is supposed to be minimized as much as possible. Furthermore, for effective treatment of a tumor, a treatment time is supposed to be minimized. Thus, the conditions for a HIFU device are: 1) a time taken to perform treatment by radiating an ultrasound wave is to be minimized; 2) a focus area of the ultrasound wave is supposed to be formed; and 3) the ultrasound wave is not supposed to be amplified except for the focus area.
Avoiding strong side lobes is very relevant also in other fields than medical treatment, such as in antenna engineering in general, especially in conjunction with multi-focus ultrasound. Consequently, the invention is described herein under reference to specific problems associated with medical applications, but the invention is applicable in more broader fields.

SUMMARY

One or more embodiments of the present disclosure relate to treatment devices using an ultrasound wave, and for instance, to treatment devices using a High Intensity Focused Ultrasound (HIFU) or more regular ultrasound wave.
According to one or more embodiments, apparatuses and methods for forming a multi-focus while removing grating lobes when a HIFU or other Ultrasound device is operated are provided.
According to one or more embodiments, methods for calculating outputs of transducer elements for forming a multi-focus while removing grating lobes are provided.
According to one or more embodiments, a computer-readable recording medium storing a computer-readable program for executing the described methods is provided.
Additional aspects will be set forth in part in the description which follows and, in part, will be apparent from the description, or may be learned by practice of the presented embodiments.
According to an aspect of the present disclosure, a method is provided according to claim 1.
According to another aspect of the present disclosure, a non-transitory computer-readable recording medium storing a computer-readable program for executing the method of forming an ultrasound focus is provided.
According to another aspect of the present disclosure, an apparatus for forming an ultrasound focus is provided according to claim 13.

## BRIEF DESCRIPTION OF THE DRAWINGS

These and/or other aspects will become apparent and more readily appreciated from the following description of the embodiments, taken in conjunction with the accompanying drawings in which:

FIG. 1 is a block diagram of an apparatus for forming a multi-focus according to an embodiment of the present disclosure;

FIG. 2 is a conceptual diagram of a transducer array of a High Intensity Focused Ultrasound (HIFU) device and a plurality of focus areas existing in a therapy area;

FIG. 3A illustrates a plurality of focal points existing in a therapy area that are input by a user, according to an embodiment of the present disclosure;

FIG. 3B illustrates optimal focus sets previously determined by an optimal focus determiner;

FIG. 3C illustrates focal points remaining by excluding focal points belonging to the optimal focus sets from the plurality of focal points of FIG. 3A;

FIG. 4 illustrates candidate points arranged in a line to select or reselect a predetermined number of focal points from among the candidate points in a candidate focus determiner;

FIG. 5 illustrates 5 candidate points randomly selected from among candidate points in the candidate focus determiner, which are represented as chromosomes in a genetics-based algorithm;

FIG. 6 illustrates a method of generating new chromosomes from combinations of existing chromosomes in the candidate focus determiner;

FIG. 7 illustrates a therapy area and a non-therapy area;

FIG. 8 is a block diagram of a focus forming determiner according to an embodiment of the present disclosure;

FIG. 9 illustrates a position vector $\gamma_n$ of a transducer element and a position vector $\gamma_m$ of an arbitrary focal point on a 3-dimensional rectangular coordinate system; and

FIG. 10 is a flowchart illustrating a method of forming a focal point for ultrasound therapy according to an embodiment of the present disclosure.

## DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

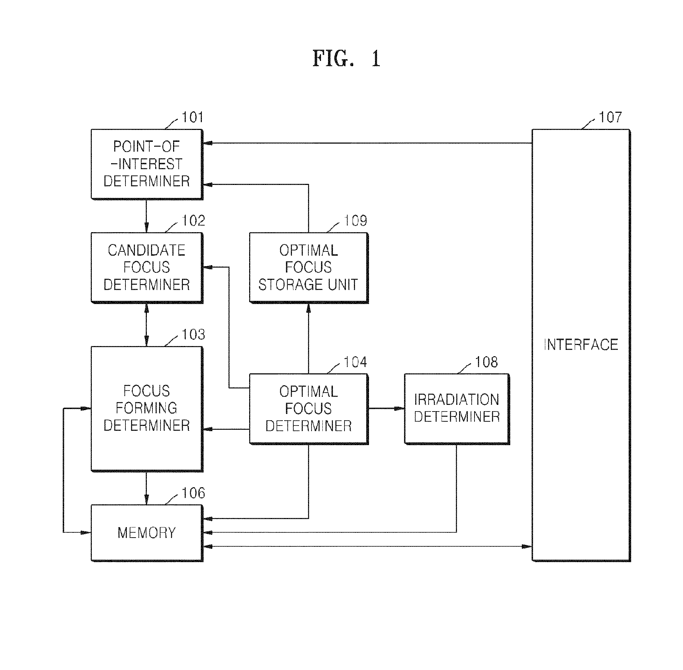

FIG. 1 is a block diagram of an apparatus for forming a multi-focus according to an embodiment of the present disclosure. Referring to FIG. 1, the apparatus may include, for example, a point-of-interest determiner 101, a candidate focus determiner 102, a focus forming determiner 103, an optimal focus determiner 104, a memory 106, an interface 107, an irradiation determiner 108, and an optimal focus storage unit 109.

When information regarding a plurality of focal points existing in a therapy area is input by a user, the point-of-interest determiner 101 determines points of interest from among the focal points and outputs the points of interest to the candidate focus determiner 102. The information regarding a plurality of focal points input by the user indicates information regarding positions of the plurality of focal points and the number of focal points in the therapy area in which a High Intensity Focused Ultrasound (HIFU) device cures a lesion by forming focal points of ultrasound waves. The phrase "points of interest," as used herein, indicates points for which calculation according to an embodiment of the present disclosure is performed, from among the plurality of focal points existing in the therapy area.

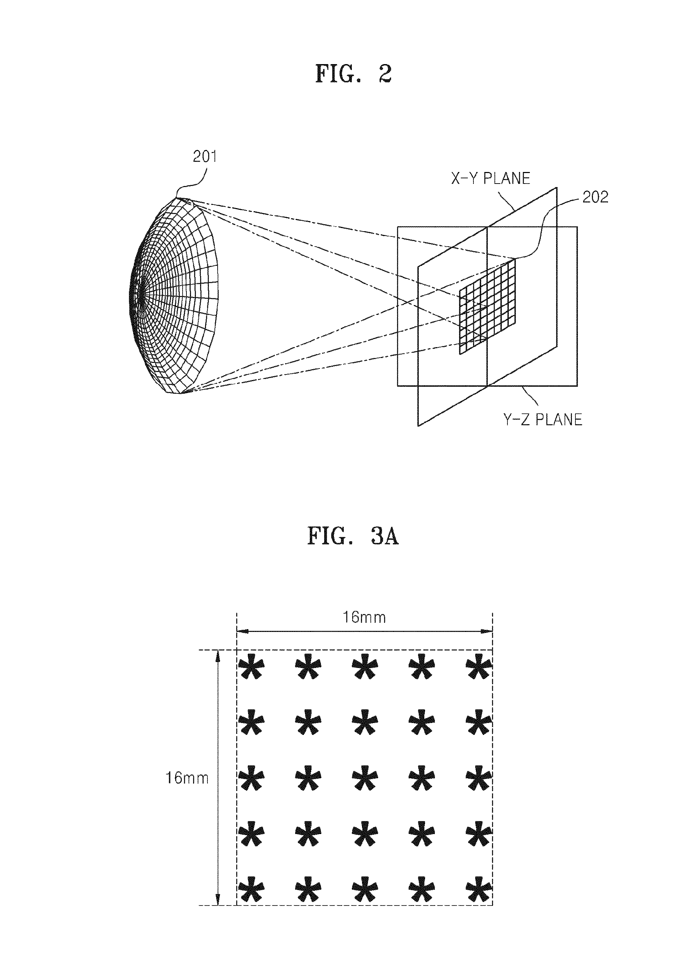

FIG. 2 is a conceptual diagram of a transducer array 201 of a HIFU device and a plurality of focus areas existing in a therapy area 202. The transducer array 201 of the HIFU device may have an antenna shape, as shown in FIG. 2, because the antenna shape makes it easier to concentrate outputs of the transducer array 201 on a predetermined area. The transducer array 201 of the HIFU device focuses ultrasound signals on the therapy area 202. The HIFU device generates vibration by providing an electrical signal to transducer elements and ultrasound waves are generated by the vibration. In this case, the HIFU device may determine a predetermined number of points of interest in the therapy area 202, based on, for example, the plurality of focal points input by the user, and may generate heat by simultaneously focusing the ultrasound waves on the predetermined number of points of interest.

FIG. 3A illustrates a plurality of focal points existing in a therapy area that are input by a user, according to an embodiment of the present disclosure. The plurality of focal points shown in FIG. 3A exists in the therapy area with 16 mm width × 16 mm length, and each focal point is indicated by *. The number of focal points is set to 25. Although in the current embodiment, focal points are described as being input for a predetermined area, focal points may equally be input by the user for a predetermined volume, as the therapy area.

When information regarding a plurality of focal points existing in a therapy area is input by the user, the point-of-interest determiner 101 immediately outputs the plurality of focal points to the candidate focus determiner 102. That is, all the focal points shown in FIG. 3A are output to the candidate focus determiner 102.

The apparatus may determine a maximum number of focus sets capable of being formed without grating lobes occurring by one-time irradiation of ultrasound waves. A process of determining a maximum number of focus sets in the apparatus will now be described in detail. First, the apparatus determines whether grating lobes occur when a single focal point is formed.

The term "grating lobes" refers to a phenomenon in which an ultrasound wave is amplified at a position other than a focus position. In more detail, an ultrasound transducer array has a structure in which a plurality of transducer elements is periodically repeated with a constant gap. When the gap between every two periodically repeated transducer elements is greater than $\lambda/2$ ($\lambda$ is a wavelength of the ultrasound wave), interference may occur between signals generated by the transducer elements, resulting in grating lobes, which describes a phenomenon in which amplification occurs in an area other than a desired focal point. In grating lobes, a magnitude of amplification in an area other than the focal point may be similar to the magnitude of amplification at the focal point. Grating lobes are an unwanted phenomenon in the HIFU device because the HIFU device generates heat by forming a focal point of an ultrasound signal at a position of a lesion to cure the lesion. However, when grating lobes occur, an ultrasound signal may be amplified at a position of a tissue or organ other than the desired position of the lesion. When the ultrasound signal is amplified in an area of the different tissue or organ, cells besides the lesion may be damaged due to heat generated by the amplified ultrasound signal. Thus, grating lobes are an unwanted phenomenon in the HIFU device and should be removed.

In an embodiment, the following technique may be employed to prevent grating lobes. After determining that grating lobes do not occur when a single focal point is formed, the apparatus may search for a focus set for which grating lobes do not occur while iteratively increasing the number of focal points by a quantity of 1, although different quantities of focal points other than 1 may alternatively be used. Assuming that grating lobes do not occur when the apparatus forms focal points for all the focal points input by the user by one-time irradiation of ultrasound waves, the apparatus may determine that ultrasound waves are irradiated one time for all the focal points.

If grating lobes will occur when the apparatus irradiates ultrasound waves simultaneously for all the focal points input by the user, the apparatus may instead sequentially perform the irradiation of ultrasound waves for some of the focal points a plurality of times, until all of the focal points input by the user have been irradiated. In this case, to find a focal point formed at each time in which the apparatus irradiates ultrasound waves, the apparatus may determine a focus set for which grating lobes do not occur when ultrasound waves are irradiated simultaneously while repeatedly increasing the number of focal points by 1.

Several focus sets are determined by repeating this determining process. When the apparatus sequentially irradiates ultrasound waves to a maximum number of focal points determined by the repeated processes, the apparatus may form focal points of ultrasound waves for all the focal points input by the user, for example, as shown in FIG. 3A. However, since the apparatus inevitably determines one focus set in a one-time operation when the apparatus determines focus sets, the apparatus determines focus sets to which ultrasound waves are sequentially irradiated through a process of repeating the same operation several times to determine several focus sets.

The point-of-interest determiner 101 determines a focus set from focal points remaining by excluding focal points included in already-determined focus sets from a previous operation. Thus, when the point-of-interest determiner 101 continuously determines points of interest, the point-of-interest determiner 101 determines, as points of interest, focal points remaining by excluding focal points belonging to an optimal focus set stored in the optimal focus storage unit 109 from among the plurality of focal points shown in FIG. 3A. The term "optimal focus set" refers to a focus set having a maximum number of focal points while still ensuring that grating lobes do not occur. The optimal focus determiner 104 determines a set of optimal focal points from among the points of interest determined by the point-of-interest determiner 101. In a successive iteration, another set of optimal focal points may be determined from among focal points remaining by excluding the already-determined focal points. Thus, the point-of-interest determiner 101 determines focal points other than the previously determined set of optimal focal points as points of interest.

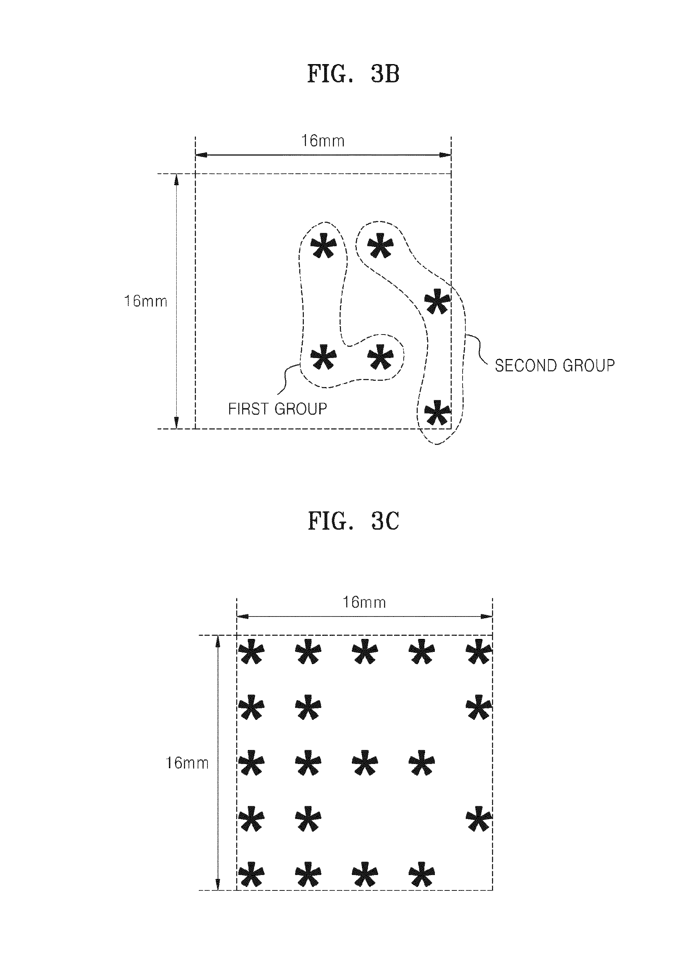

FIG. 3B illustrates optimal focus sets previously determined by the optimal focus determiner 104. A first group and a second group, as indicated in FIG. 3B, are optimal focus sets previously determined by the optimal focus determiner 104. The point-of-interest determiner 101 determines, as points of interest, focal points remaining by excluding focal points belonging to the optimal focus sets previously determined by the optimal focus determiner 104, from among the plurality of focal points shown in FIG. 3A, and outputs position and coordinate information of the determined focal points to the candidate focus determiner 102. As shown in FIG. 3B, since the first group and the second group are optimal focus sets, the point-of-interest determiner 101 excludes focal points belonging to the first group and focal points belonging to the second group from current points of interest and determines only the remaining focal points as the points of interest. FIG. 3C illustrates remaining focal points by excluding focal points belonging to the optimal focus sets from the plurality of focal points of FIG. 3A. The point-of-interest determiner 101 determines the remaining focal points, as shown in FIG. 3C, as points of interest.

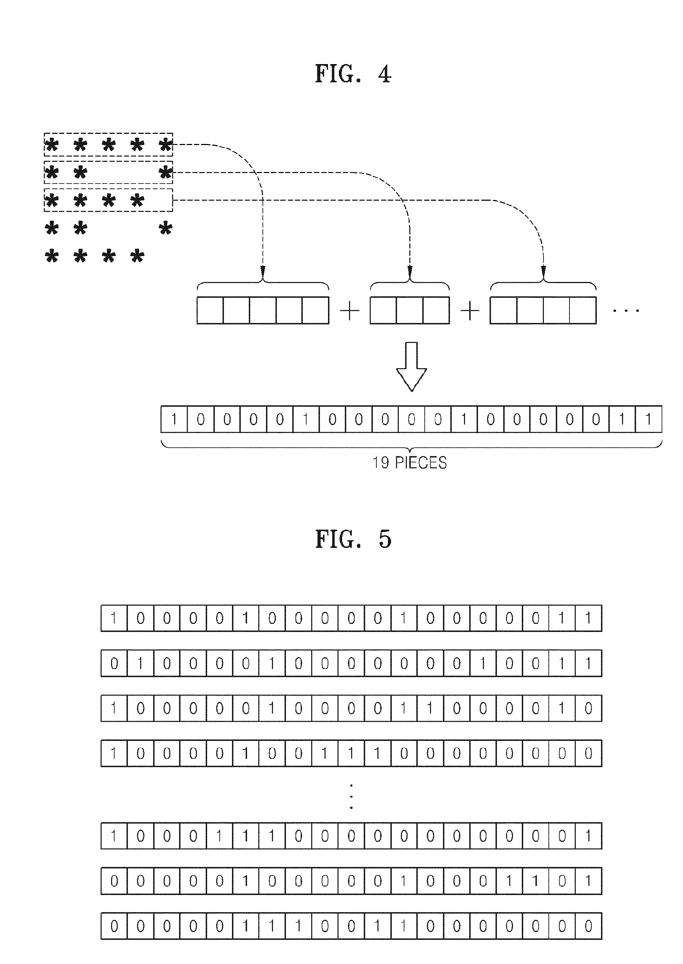

The candidate focus determiner 102 selects a number of focal points equal to the number of focal points input from the optimal focus determiner 104 from among the points of interest determined by the point-of-interest determiner 101. If the number of focal points is not input from the optimal focus determiner 104, the candidate focus determiner 102 selects points corresponding to a default number of focal points. In this case, the default number of points set in the optimal focus determiner 104 may be 2, as an example. Thus, when the number of focal points is not input from the optimal focus determiner 104, the candidate focus determiner 102 selects two focal points from among the points of interest and outputs the two selected focal points to the focus forming determiner 103. If the number of focal points is input from the optimal focus determiner 104, the candidate focus determiner 102 selects focal points corresponding to the input number of focal points from among the points of interest and outputs the selected focal points to the focus forming determiner 103. For example, if 4 is input from the optimal focus determiner 104 as the number of focal points, the candidate focus determiner 102 selects four focal points from among the points of interest and outputs the four selected focal points to the focus forming determiner 103. A method of selecting focal points from among the points of interest will be described in detail with reference to FIG. 4 below.

As such, the focal points selected by the candidate focus determiner 102 are output to the focus forming determiner 103, and the focus forming determiner 103 calculates whether the focal points selected by the candidate focus determiner 102 belong to a new optimal focus set.

If a request for reselecting focal points is input from the optimal focus determiner 104, the candidate focus determiner 102 reselects focal points and outputs the reselected focal points to the focus forming determiner 103. A reason for the optimal focus determiner 104 to request reselection may be as follows. The focus forming determiner 103 determines whether the focal points selected by the candidate focus determiner 102 are in focus without the occurrence of grating lobes. If the focus forming determiner 103 determines that the focal points selected by the candidate focus determiner 102 are in focus without the occurrence of grating lobes, the optimal focus determiner 104 requests the candidate focus determiner 102 to increase the number of focal points by 1. That is, in an embodiment the number of focal points is iteratively increased by a quantity of 1 to calculate whether an optimal focus set may be created with a greater number of focal points. If the focus forming determiner 103 determines that it is impossible that the focal points selected by the candidate focus determiner 102 are in focus without the occurrence of grating lobes, the optimal focus determiner 104 requests the candidate focus determiner 102 to reselect other focal points. That is, the optimal focus determiner 104 requests the candidate focus determiner 102 to select another focus set from among the points of interest since the selected focus set is determined as not being an optimal focus set.

When the optimal focus determiner 104 requests reselection, the number of focal points reselected by the candidate focus determiner 102 is the same as the number of focal points selected by the candidate focus determiner 102 before the reselection request. For example, if the candidate focus determiner 102 has selected 3 focal points, and the focus forming determiner 103 has calculated optimal negative pressures of the 3 focal points and derived a result indicating that grating lobes will occur, the candidate focus determiner 102 newly selects 3 different focal points in response to a reselection request and outputs the 3 selected focal points to the focus forming determiner 103.

When the candidate focus determiner 102 receives the number of focal points and a reselection request from the optimal focus determiner 104, the candidate focus determiner 102 reselects focal points corresponding to the received number of focal points from among the points of interest determined by the point-of-interest determiner 101 and outputs the reselected focal points to the focus forming determiner 103. That is, if the focus forming determiner 103 derives a result that a focus set selected by the candidate focus determiner 102 is in focus while grating lobes do not occur, the optimal focus determiner 104 requests the candidate focus determiner 102 to reselect focal points corresponding to the number of focal points increased by a quantity of 1. In an embodiment, the reselected focal points include the originally selected focus set plus an additional focal point. The reason the optimal focus determiner 104 requests the candidate focus determiner 102 to reselect focus positions even though the focus forming determiner 103 has calculated a focus set for which grating lobes do not occur is because it is desirable to maximize a quantity of focal points that are simultaneously in focus in a single ultrasound wave transmission. That is, if the focus forming determiner 103 has calculated a focus set for which grating lobes do not occur when the optimal focus determiner 104 has determined the number of focal points as 3, it is subsequently calculated whether 4 focal points are simultaneously in focus. Thus, if an optimal focus set having 3 focal points has been calculated, the focus forming determiner 103 requests the optimal focus determiner 104 to increase the number of focal points by a quantity of 1, and the optimal focus determiner 104 increases the number of focal points from 3 to 4 and simultaneously outputs the increased number of focal points and a reselection request to the candidate focus determiner 102. In an embodiment, the increased number of focal points output by the optimal focus determiner 104 includes the three focus points originally selected by the optimal focus determiner 104, plus the fourth additional focal point.

FIG. 4 illustrates points of interest arranged in a line to select or reselect a predetermined number of focal points from among the points of interest in the candidate focus determiner 102. A process of selecting focal points in the candidate focus determiner 102, according to an embodiment of the present disclosure, uses a genetic algorithm.

The genetic algorithm is a heuristic search process mimicking natural evolution. For example, instead of always randomly selecting focal points in the process of selecting focal points from among points of interest in the candidate focus determiner 102, the candidate focus determiner 102 randomly selects focal points first 10 times, sorts 5 focus sets having a better result than others from among 10 focus sets, and selects focal points by combining the 5 sorted focus sets. That is, since crossing excellent focus sets is similar to natural evolution, this is called a genetic algorithm. The phrase "excellent focus sets" as used herein may refer to focus sets having a better result than other focus sets, that is focus sets having a quantity of grating lobes that is relatively small or having relatively low intensity grating lobes compared with the other focus sets. Alternatively, "excellent focus sets" may indicate that performance is excellent when considering both the quantity and intensity of grating lobes. FIG. 4 shows points of interest arranged in a line in the candidate focus determiner 102 to apply the genetic algorithm thereto, and such a set of points of interest arranged in a line is called a chromosome. The * marks on the upper left part of FIG. 4 denote sets of points of interest shown in FIG. 3C. When the candidate focus determiner 102 arranges the sets of points of interest in a line, focal points excluded in the middle are also excluded from the arrangement. Thus, if the candidate focus determiner 102 generates a chromosome based on the case of FIG. 3C, the chromosome has 19 elements. Numbers in the chromosome denote selection or non-selection. For example, as shown in FIG. 4, in a chromosome composed of 1 and 0, 1 denotes that the candidate focus determiner 102 has selected a corresponding focal point, and 0 denotes that the candidate focus determiner 102 has not selected a corresponding focal point. Thus, since the quantity of 1s is 5 in the chromosome shown in FIG. 4, it indicates that the candidate focus determiner 102 has selected 5 focal points from among 19 points of interest as the focus set.

FIG. 5 illustrates five points of interest randomly selected from among points of interest in the candidate focus determiner 102, which are represented as chromosomes in a genetics-based algorithm. Each chromosome indicates that the candidate focus determiner 102 has selected 5 focal points from among 19 points of interest. For example, when the candidate focus determiner 102 generates 10 chromosomes in this way, 5 chromosomes having excellent performance may be sorted. In this case, "excellent performance" may indicate that the number of grating lobes is small or the intensity of grating lobes is low when 5 focal points in each of the 5 sorted chromosomes are in focus. Alternatively, the excellent performance may indicate that performance is excellent by considering both the number and intensity of grating lobes. When the 5 chromosomes having excellent performance are sorted, the candidate focus determiner 102 creates new sets using the 5 chromosomes. In this case, the 5 sorted chromosomes having excellent performance are called maternal chromosomes.

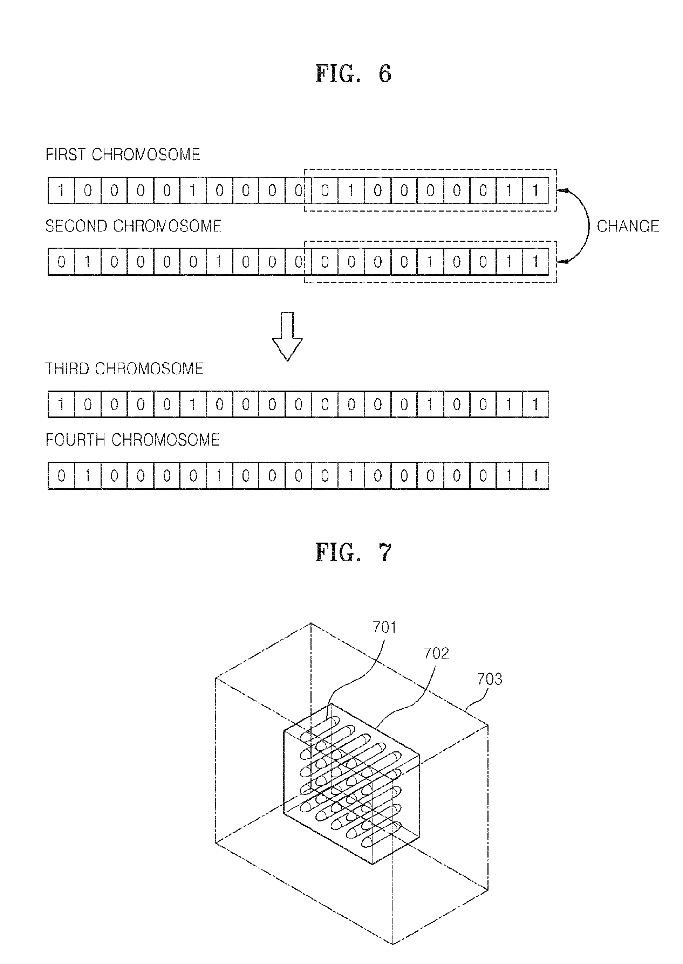

FIG. 6 illustrates a method of generating new chromosomes from combinations of existing chromosomes in the candidate focus determiner 102. A first chromosome and a second chromosome are existing chromosomes, and a third chromosome and a fourth chromosome are chromosomes newly generated by combining the first chromosome and the second chromosome in the candidate focus determiner 102 in the genetics-based algorithm. The first and second chromosomes are chromosomes calculated by the candidate focus determiner 102 as excellent chromosomes from among the chromosomes arranged in FIG. 5. When the candidate focus determiner 102 combines the first and second chromosomes, the third and fourth chromosomes are generated by exchanging some elements of the first and second chromosomes as shown in FIG. 6. In this case, the number of exchanged elements of the first chromosome is the same as the number of exchanged elements of the second chromosome. The number of exchanged elements may be predetermined by the candidate focus determiner 102 or one of 1 to the total number of elements of a chromosome. In more detail, the exchange may be performed from one element to all elements except one of each of the first chromosome and the second chromosome. In the same way, the candidate focus determiner 102 generates new chromosomes by combining maternal chromosomes, and the newly generated chromosomes are called child chromosomes. However, in this case, the number of selected focal points of a child chromosome generated by exchanging some elements of a maternal chromosome by the candidate focus determiner 102 may be different from the number of selected focal points of the maternal chromosome. Since a selected focal point is represented by the number 1, a case where the number of 1s or 0s is changed is a case where the number of selected focal points is changed. The candidate focus determiner 102 removes child chromosomes having the number of selected focal points different from that of maternal chromosomes from among all generated child chromosomes. That is, the candidate focus determiner 102 selects focal points corresponding to a predetermined number from among points of interest by using the genetic algorithm instead of random selection. When the selection is performed using the genetic algorithm, excellent chromosomes are newly combined, so a more efficient result may be derived than random selection. The candidate focus determiner 102 may infinitely generate new chromosomes by repeating the process described above, and the newly generated chromosomes are output to the focus forming determiner 103.

The focus forming determiner 103 determines whether grating lobes occur when the focal points selected by the candidate focus determiner 102 are in focus. The focus forming determiner 103 may output the determination result to the optimal focus determiner 104.

FIG. 7 illustrates a therapy area 702 and a non-therapy area 703. As an example, the therapy area 702 may be an area in which a lesion exists, and the non-therapy area 703 may be an organ area surrounding the lesion. In the therapy area 702, a plurality of focal points 701 may be formed as shown in FIG. 3A. Ideally, the H I FU device is supposed to concentrate ultrasound signals to the therapy area 702 without amplifying any ultrasound signals in the non-therapy area 703. As described above, a phenomenon that ultrasound signals are amplified in the non-therapy area 703 typically indicates the existence of grating lobes.

For example, when the focal points belonging to the first group of FIG. 3B are selected by the candidate focus determiner 102, the focus forming determiner 103 calculates a solution by which negative pressures at positions of the selected focal points are 1 and simultaneously negative pressures at some positions in the non-therapy area 703 are 0, and determines whether grating lobes occur in any part of the non-therapy area 703 when focal points are formed using the solution. A method of determining whether grating lobes occur in the focus forming determiner 103 will now be described below. It is to be emphasized, that the below provided example of a way to determine whether grating lobes occur, can be replaced by any other analysis, simulation and/or predictive model and method, which is therefore not to be interpreted as a restriction of the present invention.

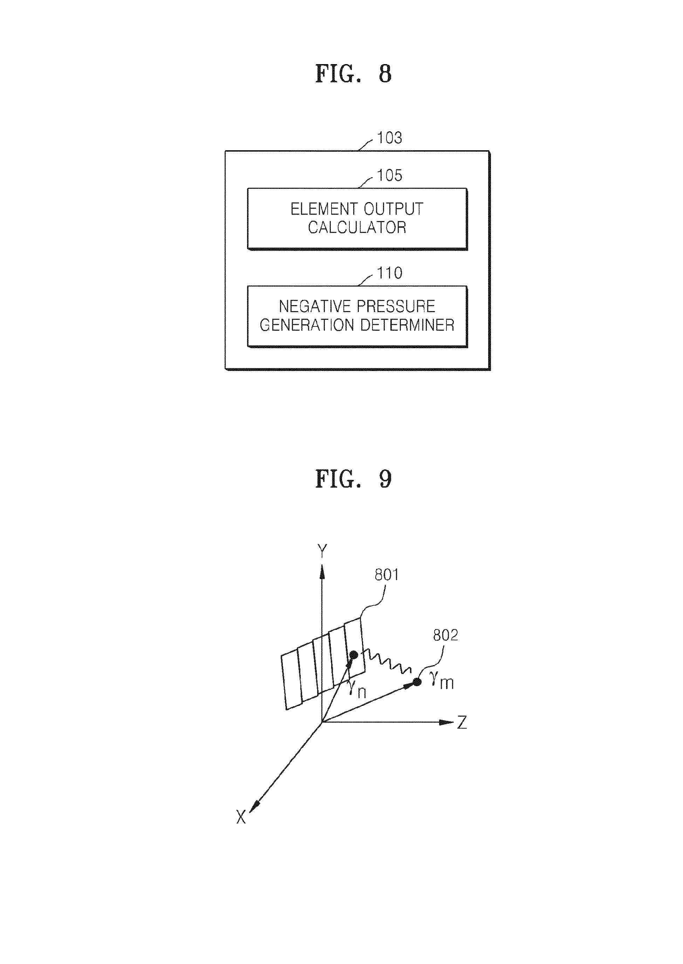

FIG. 8 is a block diagram of the focus forming determiner 103 according to an embodiment of the present disclosure. Referring to FIG. 8, the focus forming determiner 103 shown in FIG. 1 includes an element output calculator 105 and a negative pressure generation determiner 110. The element output calculator 105 calculates output of each transducer element by which candidate focal points selected by the candidate focus determiner 102 are in focus and simultaneously negative pressures of some of non-focus areas are 0. A process of calculating, by the element output calculator 105, an output by which candidate focal points selected are in focus and simultaneously negative pressures of some of non-focus areas are 0 will now be described with reference to FIG. 9. FIG. 9 illustrates a position vector $r_n$ of a transducer element 801 and a position vector $r_m$ of an arbitrary focal point 802 on a 3-dimensional (3D) rectangular coordinate system. Since the number of transducer elements is plural and positions of transducer elements are different from each other in a 3D space, to calculate a negative pressure at an arbitrary focal position, a position vector $r_n$ of each transducer element may be defined. As shown in FIG. 9, a 3D rectangular coordinate system may be set arbitrarily. It may be defined that a vector of which a starting point is the origin of the set 3D rectangular coordinate system and an ending point is a position of an nth transducer element is $r_n$, and the vector $r_n$ may be used as a position vector of the nth transducer element. Likewise, it may be defined that a vector of which a starting point is the origin of the set 3D rectangular coordinate system and an ending point is a position of an mth focal point is $r_m$, and the vector $r_m$ may be used as a position vector of the mth focal point.

Equation 1 is a fundamental expression for the element output calculator 105 to calculate a negative pressure, and an integral area in Equation 1 is called a Rayleigh-Sommerfeld integral.

Equation 1:

$$\sum_{n=1}^{N} u_n \frac{j\rho c k}{2\pi} \int_{s_n} \frac{e^{-jk|r_m - r_n|}}{|r_m - r_n|} dS_n = p(r_m)$$

Equation 1 is an expression for calculating a negative pressure $p(r_m)$ at the mth focal point.

In Equation 1, j denotes a complex number $\sqrt{-1}$, $u_n$ denotes an amplitude and phase ($|u_n| \angle \theta_n$) of a signal applied as a complex number from a transducer element, $\rho$ denotes density of air, k denotes the number of waves, c denotes a velocity of sound, N denotes the number of transducer elements, M denotes the number of points of interest, $r_m$ denotes a position vector of an mth focal point as shown in FIG. 9, $r_n$ denotes a position vector of an nth transducer element as shown in FIG. 9, and $S_n$ denotes a surface area of a transducer element. The reason the Rayleigh-Sommerfeld integral is represented in a sigma form in Equation 1 is because a negative pressure actually generated at a position of the vector $r_m$ is derived only when negative pressures generated from all transducer elements are summed up since the number of transducer elements is N.

Furthermore, to calculate negative pressures $p(\gamma_1)$, $p(\gamma_2)$, ... corresponding to a plurality of focal points in a matrix form in addition to the negative pressure at the mth focal point, $H_{(m,n)}$ may be defined as Equation 2.

Equation 2:

$$H_{(m,n)} = \frac{j\rho ck}{2\pi} \int_{S_n} \frac{e^{-jk|r_m - r_n|}}{|r_m - r_n|} dS_n$$

When H(m, n) is defined as Equation 2, negative pressures at M focal points may be represented with only one expression in a matrix form as in Equation 3 below.
Equation 3:

$$\begin{bmatrix} H_{(1,1)} & H_{(1,2)} & H_{(1,3)} & \cdots & H_{(1,n)} \\ H_{(2,1)} & H_{(2,2)} & H_{(2,3)} & \cdots & H_{(2,n)} \\ \vdots & \vdots & \vdots & \vdots & \vdots \\ H_{(m,1)} & H_{(m,2)} & H_{(m,3)} & \cdots & H_{(m,n)} \end{bmatrix} \begin{bmatrix} u_1 \\ u_2 \\ \vdots \\ u_N \end{bmatrix} = \begin{bmatrix} p(r_1) \\ p(r_2) \\ \vdots \\ p(r_M) \end{bmatrix}$$

In Equation 3, $u_1$, $u_2$, ... and $p(\gamma_1)$, $p(\gamma_2)$, ... are the same as those in Equation 1.
When Equations 4, 5, and 6 are defined as below, Equation 3 is represented as Equation 7.
Equations 4-7:

$$\begin{bmatrix} H_{(1,1)} & H_{(1,2)} & H_{(1,3)} & \cdots & H_{(1,n)} \\ H_{(2,1)} & H_{(2,2)} & H_{(2,3)} & \cdots & H_{(2,n)} \\ \vdots & \vdots & \vdots & \vdots & \vdots \\ H_{(m,1)} & H_{(m,2)} & H_{(m,3)} & \cdots & H_{(m,n)} \end{bmatrix} = H \qquad (4)$$

$$\begin{bmatrix} u_1 \\ u_2 \\ \vdots \\ u_N \end{bmatrix} = u \qquad (5)$$

$$\begin{bmatrix} p(r_1) \\ p(r_2) \\ \vdots \\ p(r_M) \end{bmatrix} = p \qquad (6)$$

$$Hu = p \qquad (7)$$

Equation 7 is the same expression as Equation 3 but is more simply represented by defining a specific matrix in Equations 4, 5, and 6. That is, **p** represents negative pressures at focus positions $r_1$ to $r_m$ as an (M×1) matrix as represented in Equation 6, and in the case of FIG. 3A, M=25. In addition, **H** denotes an (MxN) matrix indicating the arrangement of Equation 2 that is defined in Equation 4, and **u** denotes an (N×1) matrix indicating amplitudes and phases of signals applied to the N transducer elements.

The element output calculator 105 sets negative pressures of focal points selected in the focus area 702 by the candidate focus determiner 102 as 1, sets negative pressures of focal points not selected in the focus area 702 by the candidate focus determiner 102 as 0, and derives a value of **u** satisfying Equation 7. The number of values of **u** may be plural instead of singular because the number of unknowns to be determined is greater than the number of simultaneous equations if N>M when the number of unknowns to be determined is N, which is the number of transducer elements, and the number of simultaneous equations generated by Equation 7 is M. Thus, the focus forming determiner 103 searches for solutions satisfying a condition that grating lobes do not occur from among a plurality of solutions satisfying Equation 7. Equation 7 may be represented as Equation 8 when linear algebra is used.

Equation 8:

$$u = H^+ p + \left(1 - H^+ H\right) w$$

In Equation 8, **w** denotes an arbitrary vector, and a superscript '+' denotes a pseudo inverse matrix.

Although an arbitrary value may be designated to the vector **w** since the vector **w** is an arbitrary vector, a value by which grating lobes do not occur in the non-therapy area 703 of FIG. 7 is calculated. That is, the vector **w** satisfying a condition that negative pressures in the non-therapy area 703 are 0 is calculated. However, since infinite points exist in the non-therapy area 703, it is difficult to represent a condition that negative pressures in the entire non-therapy area 703 are 0 with an equation. Thus, an equation is created to make negative pressures of a predetermined number of points randomly selected in the non-therapy area 703 zero (0) or make negative pressures of parts to be protected because of weakness against heat zero (0), and the vector **w** satisfying the created equation is calculated. An example in which negative pressures in a protected area are made 0 will now be described. When the number of positions in the protected area is C, the negative pressures in the protected area may be represented with a matrix as Equation 9, and a condition that all negative pressure values at the positions are 0 is the condition that grating lobes do not occur.

Equation 9:

$$\begin{bmatrix} p(r'_1) \\ p(r'_2) \\ \vdots \\ p(r'_C) \end{bmatrix} = p' = \begin{bmatrix} 0 \\ 0 \\ \vdots \\ 0 \end{bmatrix}$$

In Equation 9, a superscript ' is attached to **p** to identify the negative pressures in the protected area. A matrix **H**(m, n) in the protected area may be represented as **B** as Equation 10 to discriminate it from focus areas. In Equation 10, a superscript "' of H indicates that the matrix H(m, n) is in the protected area.
Equation 10:

$$\begin{bmatrix} H'_{(1,1)} & H'_{(1,2)} & H'_{(1,3)} & \cdots & H'_{(1,n)} \\ H'_{(2,1)} & H'_{(2,2)} & H'_{(2,3)} & \cdots & H'_{(2,n)} \\ \vdots & \vdots & \vdots & \vdots & \vdots \\ H'_{(C,1)} & H'_{(C,2)} & H'_{(C,3)} & \cdots & H'_{(C,n)} \end{bmatrix} = B$$

Equation 11 may be obtained using Equations 9 and 10.
Equation 11:

$$Bu = 0$$

Equation 11 shows a condition that all negative pressures in the protected area are 0. When the element output calculator 105 calculates values of **u** satisfying both Equations 7 and 10, ultrasound waves may be focused on a desired focal point according to Equation 7, and negative pressures in the protected area may be removed according to Equation 11. When the element output calculator 105 substitutes the values of **u** into Equation 11 to calculate a solution of Equation 11, Equation 12 may be obtained.
Equation 12:

$$Bu = B \times \left( H^+ p + \left( 1 - H^+ H \right) w \right) = 0$$

A solution of **w** in Equation 12 is obtained as Equation 13 by linear algebra. The value of **w** in Equation 13 indicates a condition that negative pressures in the protected area are 0, i.e., a condition that grating lobes do not occur in the protected area.
Equation 13:

$$w = \left( B\left(1 - H^+ H\right)\right)^+ \left( - BH^+ p\right)$$

When values of the vector **w are** calculated, the element output calculator 105 substitutes the values of the vector **w** into Equation 8 to calculate values of **u** that are output values of transducer elements and stores the calculated values of **u** in the memory 106 in correspondence with input candidate focal points. Since the calculated values of **u** satisfy both Equations 7 and 11, the calculated values of **u** are output values of transducer elements, which satisfy that the candidate focal points are in focus and simultaneously negative pressures in the protected area are 0. The calculated outputs **u** of transducer elements are output to the negative pressure generation determiner110.

The negative pressure generation determiner 110 determines whether grating lobes occur in the entire non-focus area when the outputs **u** of transducer elements that are calculated by the element output calculator 105 are applied to the transducer elements. The element output calculator 105 has calculated **u** under the condition that negative pressures in the protected area are 0 so that grating lobes do not occur in the non-focus area, and particularly, in the protected area. Thus, when the outputs **u** calculated by the element output calculator 105 are used, grating lobes do not occur in the protected area. However, since grating lobes may occur in the entire non-focus area other than the protected area, the negative pressure generation determiner 110 determines whether grating lobes occur in the entire non-focus area. Furthermore, in a case where negative pressures of grating lobes are low or a case where grating lobes occur in an area harmless to a human body, the negative pressure generation determiner 110 may determine that grating lobes do not occur. The negative pressure generation determiner 110 determines whether grating lobes occur and outputs a determination result to the optimal focus determiner 104.

When the optimal focus determiner 104 receives a determination result that grating lobes occur from the negative pressure generation determiner 110, the optimal focus determiner 104 outputs the same quantity of focal points as before to the candidate focus determiner 102 and requests the candidate focus determiner 102 to reselect focal points corresponding to the quantity. A focus set reselected by the candidate focus determiner 102 is calculated again by the focus forming determiner 103 based on whether grating lobes occur. Even though the candidate focus determiner 102 may repeatedly reselect focal points up to a predetermined number of times, if grating lobes continuously occur, the optimal focus determiner 104 stops requesting the candidate focus determiner 102 for reselection of focal points. That is, the optimal focus determiner 104 determines that grating lobes will continuously occur with the existing number of focal points and stops requesting reselection of focal points. For example, even though the optimal focus determiner 104 has continuously requested the candidate focus determiner 102 to select 7 focal points, if grating lobes still occur within, for example, 50 times, the optimal focus determiner 104 determines that the occurrence of grating lobes cannot be removed with 7 focal points and does not further request the candidate focus determiner 102 to reselect another 7 focal points.

When the optimal focus determiner 104 stops requesting the candidate focus determiner 102 to reselect focal points, the optimal focus determiner 104 determines that the latest focus set stored in the memory 106 is an optimal focus set. In the memory 106, a focus set for which grating lobes do not occur is stored as a focus set. The quantity of focal points in the stored focus set is one less than the number of focal points for which the reselection request has been requested. The optimal focus determiner 104 then outputs **u** of transducer elements corresponding to the stored focus set. That is, for example, after the optimal focus determiner 104 determines that the occurrence of grating lobes cannot be removed with 7 focal points, the optimal focus determiner 104 determines that a focus set made up of 6 focal points, which is stored in the memory 106, is an optimal focus set. This focus set is a focus set for which grating lobes do not occur. As such, the optimal focus set determined by the optimal focus determiner 104 is stored as an optimal focus set in the optimal focus storage unit 109 together with values of u.

When the optimal focus determiner 104 receives a determination result from the focus forming determiner 103 that grating lobes do not occur for the present number of focal points, the optimal focus determiner 104 outputs a quantity of focal points equal to the present number of focal points plus 1 focal point to the candidate focus determiner 102 and requests the candidate focus determiner 102 to select focal points corresponding to the output number. That is, a present focus set indicates a set of focal points capable of being in focus without the occurrence of grating lobes. However, to determine whether grating lobes may or may not occur with even an increased quantity of focal points, the optimal focus determiner 104 requests the candidate focus determiner 102 to select more focal points.

As described above, the point-of-interest determiner 101 excludes an optimal focus set received from the focus forming determiner 103 when points of interest have been determined. If there are no further focal points when the point-of-interest determiner 101 excludes optimal focus sets from the plurality of focal points 701 existing in the therapy area 702, which are received from the user, the operation ends.

The memory 106 stores focus sets for which grating lobes do not occur from among focus sets calculated by the focus forming determiner 103 and stores values of **u** corresponding to the stored focus sets, which are calculated by the element output calculator 105.

The optimal focus storage unit 109 stores focus sets determined as optimal focus sets by the optimal focus determiner 104 and values of **u** corresponding to the focus sets.

The interface 107 allows the user to input information regarding focal points into the point-of-interest determiner 101 and outputs an optimal focus set and values of **u** corresponding to the optimal focus set, which are stored in the optimal focus storage unit 109.

The irradiation determiner 108 determines a sequence or time for irradiating ultrasound waves to focal points determined as optimal focal points by the optimal focus determiner 104. The number of sets of focal points determined as optimal focal points by the optimal focus determiner 104 may be plural. Thus, when the plurality of sets of focal points are irradiated, the irradiation determiner 108 determines a sequence or time for irradiating ultrasound waves to the plurality of sets of focal points.

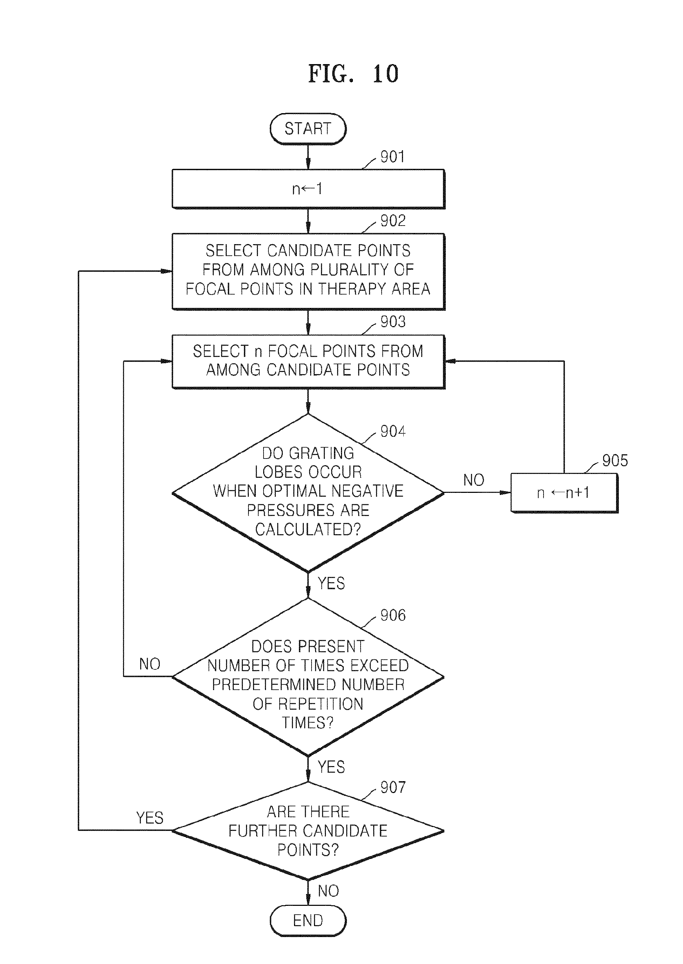

FIG. 10 is a flowchart illustrating a method of forming a focal point for ultrasound therapy according to an embodiment of the present disclosure. Referring to FIG. 10, in operation 901, the number of focal points to be determined by the candidate focus determiner 102 is set to 1. In operation 902, the point-of-interest determiner 101 selects, as points of interest, focal points remaining by excluding focal points received as an optimal focus set from the optimal focus determiner 104 from among a plurality of focal points in a therapy area, which are input by the user. In operation 903, the candidate focus determiner 102 selects n candidate focal points from among the points of interest. In operation 904, the focus forming determiner 103 calculates optimal negative pressures of the n candidate focal points selected by the candidate focus determiner 102, and if grating lobes occur, the operation proceeds to operation 906, and if grating lobes do not occur, the operation proceeds to operation 905. In operation 905 in which grating lobes do not occur, the optimal focus determiner 104 increases 1 to n+1, which is the number of focal points to be determined by the candidate focus determiner 102, and the operation proceeds to operation 903 to repeat the above process. In operation 906 in which grating lobes occur, the optimal focus determiner 104 determines whether the present number of times exceeds the predetermined number of repetition times. If the present number of times exceeds the predetermined number of repetition times, the operation proceeds to operation 907, and if the present number of times does not exceed the predetermined number of repetition times, the operation proceeds to operation 903 to repeat the above process using different focal points from among the candidate focal points. In operation 907, the point-of-interest determiner 101 determines whether there are further points of interest. If there are no further points of interest, the operation ends, and if there are further points of interest, the operation proceeds to operation 902 to repeat the above process.

As described above, according to the one or more of the above embodiments of the present disclosure, by simultaneously forming multiple focal points while removing grating lobes in an operation of a HIFU device, a therapy time of the HIFU device may be reduced, and a damage to organs around a lesion during therapy may be prevented. In addition, outputs of transducer elements for simultaneously forming a several number of focal points while removing the grating lobes may be calculated.

The embodiments of the present disclosure may be written as computer programs and may be implemented in general-use digital computers that execute the programs using a non-transitory computer-readable recording medium. In addition, a data structure used in the embodiments of the present disclosure may be recorded in the non-transitory computer-readable recording medium in various ways.

Examples of the non-transitory computer-readable recording medium include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described embodiments, or vice versa.

Any one or more of the method steps which can be embodied as software modules described herein may be executed by a controller such as a dedicated processor unique to that unit or by a processor common to one or more of the modules. The described methods may be executed on a general purpose computer or processor or may be executed on a particular machine such as the apparatus for forming a focal point for ultrasound therapy described herein.

While the present disclosure has been particularly shown and described with reference to exemplary embodiments of the present disclosure, it will be understood by those of ordinary skill in the art that various changes in form and details may be made therein without departing from the scope of the present disclosure as defined by the following claims. The exemplary embodiments should be considered in descriptive sense only and not for purposes of limitation. Therefore, the scope of the present disclosure is defined not by the detailed description of the present disclosure but by the appended claims, and all differences within the scope will be construed as being included in the present disclosure.

**Claims**

1. A method of forming an ultrasound focus, the method comprising:

   selecting at least one candidate focus position, from among focus positions, as an area to which an ultrasound wave is to be radiated;
   determining, when a focal point of the ultrasound wave is formed at the selected at least one candidate focus position, whether a negative pressure increase occurs at other positions than the at least one selected candidate focus position; and
   determining, based on a result of the determining of whether the negative pressure increase occurs, a set of candidate focus positions at which the negative pressure increase does not occur at other positions than the selected at least one candidate focus position.

2. The method of claim 1, further comprising, when it is determined that the negative pressure increase does occur, changing the candidate focus position and reselecting the changed candidate focus position.

3. The method of claim 1 or 2, further comprising, when it is determined that the negative pressure increase does not occur, changing the candidate focus position and a quantity of candidate focus positions and reselecting the changed candidate focus positions.

4. The method of claim 1, 2 or 3, further comprising determining at least one of a sequence and a time for irradiating ultrasound waves to the determined set of candidate focus positions.

5. The method of any one or more than one of the preceding claims, wherein the determining whether a negative pressure increase occurs further comprises:

   calculating outputs of transducer elements for which negative pressures of the candidate focus positions have a first value and negative pressures of positions outside a target area have a second value; and
   determining whether an increase occurs in the negative pressures of the positions outside the target area.

6. The method of claim 5, wherein each of the calculated outputs is based on one of an amplitude and phase of an electrical signal applied to each of the transducer elements.

7. The method of claim 5 or 6, wherein the determining of whether the increase occurs in the negative pressures of the positions outside the target area comprises:

   obtaining a plurality of sets of outputs of transducer elements for which negative pressures of the candidate focus positions have the first value; and
   selecting an output set for which negative pressures of positions outside the target area have the second value from among the obtained plurality of sets of outputs of transducer elements.

8. The method of any one or more than one of the preceding claims, wherein the negative pressure increase indicates a grating lobe.

9. The method of any one or more than one of the preceding claims, wherein the selecting comprises selecting the at least one candidate focus position using a genetic algorithm.

10. The method of any one or more than one of the preceding claims, further comprising subsequently determining an additional set of candidate focus positions at which the negative pressure increase does not occur at other positions than the selected at least one candidate focus position, wherein preceding selecting of the at least one candidate focus position comprises not selecting focus positions included in the previously determined set of candidate focus positions.

11. The method of any one or more than one of the preceding claims, comprising:

   calculating outputs of transducer elements for which negative pressures at focus positions upon which ultrasound waves are irradiated have a first value; and
   selecting outputs of transducer elements, from among the calculated outputs, for which negative pressures in a predetermined area other than the focus positions have a second value, if the second value is less than the first value.

**12.** A non-transitory computer-readable recording medium storing a computer-readable program for executing the method according to any one or more than one of the preceding claims.

**13.** An apparatus for forming an ultrasound focus, the apparatus comprising:

a candidate focus determiner arranged to select at least one candidate focus position, from among focus positions, as an area to which an ultrasound wave is to be radiated;
a focus forming determiner arranged to determine, when a focal point of the ultrasound wave is formed at the selected candidate focus position, whether a negative pressure increase occurs at positions other than the selected candidate focus position; and
an optimal focus determiner arranged to determine, based on a determination result of the focus forming determiner, a set of candidate focus positions at which the negative pressure increase does not occur.

**14.** The apparatus of claim 13, wherein, when the focus forming determiner determines that the negative pressure increase does occur, the optimal focus determiner is arranged to transmit a reselection request to the candidate focus determiner, and
when the reselection request is received by the candidate focus determiner , the candidate focus determiner is arranged to change the candidate focus position and to reselect the changed candidate focus position.

FIG. 1

# FIG. 2

# FIG. 3A

## FIG. 3B

16mm

16mm

FIRST GROUP

SECOND GROUP

## FIG. 3C

16mm

16mm

# FIG. 4

| 1 | 0 | 0 | 0 | 0 | 1 | 0 | 0 | 0 | 0 | 0 | 1 | 0 | 0 | 0 | 0 | 0 | 1 | 1 |

19 PIECES

# FIG. 5

| 1 | 0 | 0 | 0 | 0 | 1 | 0 | 0 | 0 | 0 | 0 | 1 | 0 | 0 | 0 | 0 | 0 | 1 | 1 |

| 0 | 1 | 0 | 0 | 0 | 0 | 1 | 0 | 0 | 0 | 0 | 0 | 0 | 0 | 1 | 0 | 0 | 1 | 1 |

| 1 | 0 | 0 | 0 | 0 | 0 | 1 | 0 | 0 | 0 | 0 | 1 | 1 | 0 | 0 | 0 | 0 | 1 | 0 |

| 1 | 0 | 0 | 0 | 0 | 1 | 0 | 0 | 1 | 1 | 1 | 0 | 0 | 0 | 0 | 0 | 0 | 0 | 0 |

| 1 | 0 | 0 | 0 | 1 | 1 | 1 | 0 | 0 | 0 | 0 | 0 | 0 | 0 | 0 | 0 | 0 | 0 | 1 |

| 0 | 0 | 0 | 0 | 0 | 1 | 0 | 0 | 0 | 0 | 0 | 1 | 0 | 0 | 0 | 1 | 1 | 0 | 1 |

| 0 | 0 | 0 | 0 | 0 | 1 | 1 | 1 | 0 | 0 | 1 | 1 | 0 | 0 | 0 | 0 | 0 | 0 | 0 |

# FIG. 6

FIRST CHROMOSOME

| 1 | 0 | 0 | 0 | 0 | 1 | 0 | 0 | 0 | 0 | 0 | 1 | 0 | 0 | 0 | 0 | 0 | 1 | 1 |

SECOND CHROMOSOME

| 0 | 1 | 0 | 0 | 0 | 0 | 1 | 0 | 0 | 0 | 0 | 0 | 0 | 0 | 1 | 0 | 0 | 1 | 1 |

CHANGE

THIRD CHROMOSOME

| 1 | 0 | 0 | 0 | 0 | 1 | 0 | 0 | 0 | 0 | 0 | 0 | 0 | 0 | 1 | 0 | 0 | 1 | 1 |

FOURTH CHROMOSOME

| 0 | 1 | 0 | 0 | 0 | 0 | 1 | 0 | 0 | 0 | 0 | 1 | 0 | 0 | 0 | 0 | 0 | 1 | 1 |

# FIG. 7

# FIG. 8

# FIG. 9

# FIG. 10

START

901
n←1

902
SELECT CANDIDATE POINTS FROM AMONG PLURALITY OF FOCAL POINTS IN THERAPY AREA

903
SELECT n FOCAL POINTS FROM AMONG CANDIDATE POINTS

904
DO GRATING LOBES OCCUR WHEN OPTIMAL NEGATIVE PRESSURES ARE CALCULATED?

NO → 905 n ←n+1

YES

906
DOES PRESENT NUMBER OF TIMES EXCEED PREDETERMINED NUMBER OF REPETITION TIMES?

NO

YES

907
ARE THERE FURTHER CANDIDATE POINTS?

YES

NO

END